# EUROPEAN PATENT SPECIFICATION

(11) **EP 4 090 729 B1**
(45) Date of publication and mention of the grant of the patent: **18.10.2023**
(21) Application number: 21700352.4
(22) Date of filing: 13.01.2021
(51) Int. Cl.: C12M 1/00, C12M 1/107, C12M 1/26, C12M 1/34, C12M 1/33

(54) **METHOD FOR PRODUCING BIOPRODUCTS FRONT STREAMS OF ORGANIC MATERIAL**
VERFAHREN ZUR HERSTELLUNG VON BIOPRODUKTFRONTSTRÖMEN VON ORGANISCHEM MATERIAL
PROCÉDÉ DE PRODUCTION DE BIOPRODUITS À PARTIR DE FLUX DE MATIÈRE ORGANIQUE

(30) Priority: 15.01.2020 BE 202005024
(43) Date of publication of application: 23.11.2022
(73) Proprietor: Dranco, Naamloze Vennootschap, 9000 Gent (BE)
(72) Inventor: GILDEMYN, Sylvia Marie-Pascale G, 9040 Sint-Amandsberg (BE); SABBE, Kevin, 9000 Gent (BE); VELGHE, Filip, Lichtervelde 8810 (BE)
(74) Representative: Jacobs, Tinneke Ivonne C
(86) International application number: PCT/IB2021/050202
(87) International publication number: WO 2021/144696

(56) References cited:
- EP-A2- 0 521 685
- WO-A1-2014/062137
- DE-A1- 19 502 856
- DE-A1-102007 061 137
- DE-B3-102008 042 461
- US-A1- 2006 266 703
- US-A1- 2009 095 673

## Description

The present invention relates to a method for producing bioproducts from streams of organic material.

In particular, the invention is intended for converting organic waste streams and energy crops, such as corn and rapeseed, into high-quality proteins.

It is known that in the past biological waste usually ended up on a landfill or was processed in an incinerator, but that now more sustainable techniques are available for processing said waste stream.

US 2009/095673 discloses a method for combining and converting solid and liquid organic waste materials to useful bioproducts. An obtained liquid fraction from a dewatering step may be added to the biomaterial as dilutant before the anaerobic digestion starts.

Currently, biological waste is used chiefly as substrate for producing compost or biogas. However, yet other methods are possible to upgrade biological waste to bioproducts with a higher added value. Thus, the waste stream can be converted into microbial protein, which can then serve as a local source of protein for the agricultural sector. Such protein production makes local agriculture more sustainable and less dependent on the import of protein-rich raw materials.

Also, this strongly expands the range of substrates suitable for biogas production, for example for a dry anaerobic fermentation process. Organic streams which initially were unsuitable for dry anaerobic digestion due to their too high fluid and/or nitrogen content are dewatered during the protein production process. The resulting dry fraction is better suited for biogas production hereafter. The liquid fraction serves as suitable organic stream for protein production. The new process, described in the present invention, is therefore directly integratable without any problem with already existing and future dry anaerobic fermentation plants.

The method for processing organic waste streams differs from country to country. The European Union (EU) produces between 118 and 138 million tonnes of biological waste annually. Only 30 million tonnes of said waste stream is sorted and upgraded by composting and digesting (European Commission, 2010, Communication from the Commission to the Council and the European Parliament on future steps in bio-waste management in the European Union, Brussels; Siebert, 2016, Bio-Waste Recycling in Europe against the backdrop of the circular economy package. Bochum, Germany). Outside the European Union too, like in cities of the global south, 80% of domestic waste comprises food leftovers and other organic waste.

Annually, 30% of the worldwide food production is wasted, the bulk of which ends up on landfills (Rezaei & Liu, 2017 Food loss and waste in the food supply chain). The best possible processing of said waste stream must be aspired to by investing in prevention and reduction of waste and the development of innovative methods for reuse and energy recovery. The current state of the art which is applied is very regionally bound and varies from dumping without energy recovery to recycling by composting and biogas production. In addition to organic waste streams, energy crops such as corn, rapeseed, sorghum and sunflower are also used as substrate for producing biogas.

The invention relates to a method for producing bioproducts from streams of organic material, which at least consists of the following steps:
(i) physical-biological pre-treatment of the organic stream to release a large amount of organic compounds from said stream.

This pre-treatment initially comprises a mixing whereby the substrate is mixed with water into a diluted stream with a sufficiently low dry matter content, more specifically a dry matter content lower than 15%, such that the pumpability of the mixture is guaranteed. One or more mechanical steps are applied hereby such as continuous grinding, blixing and continuous extruding.

The water quantity is also further adjusted such that the desired concentration of dissolved organic compounds is obtained. Concentrations between 10 and 200 grammes of dissolved chemical oxygen consumption (COC) per litre are desired, more specifically between 10 and 50 grammes per litre or between 50 and 100 grammes per litre or between 100 and 150 grammes per litre or between 150 and 200 grammes per litre COC. COC is a measure for the quantity of organic carbon present in the aqueous phase and refers specifically to the quantity of oxygen necessary to completely oxidise organic carbon into carbon dioxide. These organic compounds consist of, among others, easily soluble organic acids.

The release of organic compounds is stimulated by one or more mechanical steps and can occur both in a separate step on the fresh substrate and/or during the mixing with fresh water and process water. The mechanical steps can consist of continuous grinding with a grinding mill, blixing, continuous extruding or a mechanical treatment whereby the structure of the substrate is destroyed. The mechanical treatment during mixing can thus consist of mixing the substrate and water in a rotating drum, possibly with fixed baffles and/or heavy loose objects which result in a better mixing and rolling of the substrate.
(ii) Adding an alkaline solution to the mixture until the pH value of the mixture has reached a level between pH 5 and pH 9 to stimulate the release of these organic compounds. More specifically, said pH value can be between pH 5 and 7 or between pH 7 and 9.

The alkaline solution contains a sodium hydroxide solution of 1 to 3 mol per litre and is added once-only at the start of the mixing, such that a pH value within the aforementioned pH ranges is obtained.

Adding said alkaline solution or not is optional and depends on the desired concentration of dissolved compounds.
iii) adding an external inoculum or not consisting of a microbiome that stimulates the release of compounds during an anaerobic process. This inoculum relates to a natural anaerobic culture, for example from a digestion plant, from fermentation processes in the food industry, from the fermented food products itself such as yoghurt or from strains already present in the waste stream.

When mixing the organic stream with water, possibly with addition of the alkaline solution and/or inoculum, a contact time of five minutes to two weeks must be maintained, more specifically a contact time between five minutes and one hour or between one and six hours or between six hours and one day or between one day and one week, or between one week and two weeks. The temperature imposed during the contact time is between 10 and 80 °C, more specifically between 10 and 25 °C, or between 25 and 40 °C, or between 40 and 60 °C, or between 60 and 80 °C.

iv) Following pre-treatment, the mixture is split into a fibre fraction and a liquid fraction during a separation step. This separation can be done by a centrifugation step, compressing or another method whereby water is released from the organic fraction. At this point the fibre fraction has a sufficiently low dry matter (DM) content between 10 and 50% such that said fibre fraction is suitable for the further processing via dry digestion during a continuous process. This creates biogas and digestate as end products.

v) The liquid fraction which was released during the separation step has very low suspended matter contents, somewhere between 0 and 2.5%, and contains essentially dissolved organic compounds between 70 and 100%. Said fraction undergoes a continuous aerobic treatment whereby the organic compounds are microbiologically converted into a protein-rich product. Said conversion requires a continuous aeration, a mesophilic reactor temperature and a short hydraulic residence time without biomass-retention.

To realise an optimum conversion of the organic compounds, the necessary nutrients, such as nitrogen (N) in the form of ammonium chloride or urea and phosphorus (P) in the form of potassium or ammonium dihydrogen phosphate or ammonium phosphate are added to the reactor during said aerobic step. A COC:N:P ratio of 100:5:1 needs to be achieved hereby. Said aerobic conversion converts the present dissolved organic compounds and nutrients for at least 95% such that the remaining effluent is poor in organic compounds and nutrients.

The protein production amounts to between 125 and 275 mg/g COC, but can be between 125 and 200 or between 200 and 275 mg/g COC for example, whereby the formed biomass (as TSS) consists of 50 to 70% and sometimes up to 90% proteins. The effluent of said aerobic step is subjected to a next separation step.
vi) in a next separation step, the formed protein-rich product is separated from the liquid phase at a relative centrifugal force between 4,500 and 5,000 g or by filtration.

vii) The liquid phase is partially recirculated as process water to the mixing step and partially discharged and treated in a waste water purification plant.

(viii) The separated protein-rich product still has a too low DM content between 4 and 30% and needs to be dewatered during a drying step. Drying can take place in a kiln at temperatures between 50 and 80°C, by spray drying, or similar drying techniques whereby the quality of the end product is not affected. After drying, the end product has a final DM content of 50-85%.

An advantage of this method is that in addition to recovering compost and biogas from organic waste streams, it also allows a protein-rich bioproduct to be recovered that can be reutilised locally in agriculture and cattle breeding, for example as animal feed.

Another advantage is that the remaining waste to be dumped is limited.

Yet another advantage consists in that said method can be integrated with existing waste treatment processes such as composting and digesting, such that the processing can be limited to one process run and this preferably in a continuous process.

### Example I - Organic waste

Vegetable, fruit, and garden waste with a dry matter (DM) content of 30% were used as substrate, the garden waste fraction of which amounts to approximately 10%. This substrate initially had a biogas production potential (BPP) of 11.9 NL/kg. This substrate underwent a pre-treatment whereby the waste stream was mixed with water such that a dry matter content of 8% was obtained.

After the pre-treatment, the liquid fraction of the dry fraction was separated by means of compressing, followed by a centrifugation step at a relative centrifugal force of 4800 g for 6 minutes. The resulting dry fraction had a BPP of 1.08 NL/kg or 91% of the original BPP and a dry matter content of 40%.

The obtained liquid fraction contained 20.9 grammes total COC per litre. During the aerobic treatment of said stream, 8.6 grammes of protein-rich biomass per litre was formed with a protein content of 52.7%. This corresponds with a protein yield of 218 mg/g COC. This yield was separated by means of a centrifugation step, also at a relative centrifugal force of 4800 g for 6 minutes. After separating the supernatant, the protein-rich biomass remained as pellet. This contained a dry matter content of 15.51% and a protein content of 53% of the TSS-content.

### Example II - Energy crop

Corn-silage was used as substrate with a dry matter content of 35% and a biogas production potential of 188 NL/kg. This underwent a pre-treatment whereby the organic stream was mixed with water such that a dry matter content of 8% was obtained and a biogas production potential of 107 NL/kg. Subsequently an alkaline sodium hydroxide solution was added to the mixture until a pH of 6.5 was reached, after which the mixture was incubated at 37 °C for four days. After the pre-treatment the liquid fraction was separated from the dry fraction by means of compressing, followed by a centrifugation step at a relative centrifugal force of 4800 g for 6 minutes. The resulting dry fraction had a BPP of 107 NL/kg and a dry matter content of 45%. The obtained liquid fraction contained 31.7 grammes total COC per litre. During the aerobic treatment of said stream, 8.5 grammes of protein-rich biomass per litre was formed with a protein yield of 168 mg/g COC. This was separated by means of a centrifugation step, also at a relative centrifugal force of 4800 g for 6 minutes. After separating the supernatant, the protein-rich biomass remained as pellet. Said biomass contained a dry matter content of 4.80% and a protein content of 63% of the TSS-content.

In one run, the method also produces biogas and digestate as bioproducts, which can be used as fuel and as compost for fertilising and enhancing farmland.

With the intention of better showing the characteristics of the invention, a preferred application of the method for the production of bioproducts from streams of organic material according to the invention is described hereinafter, by way of an example without any limiting nature, with reference to the accompanying drawings wherein:

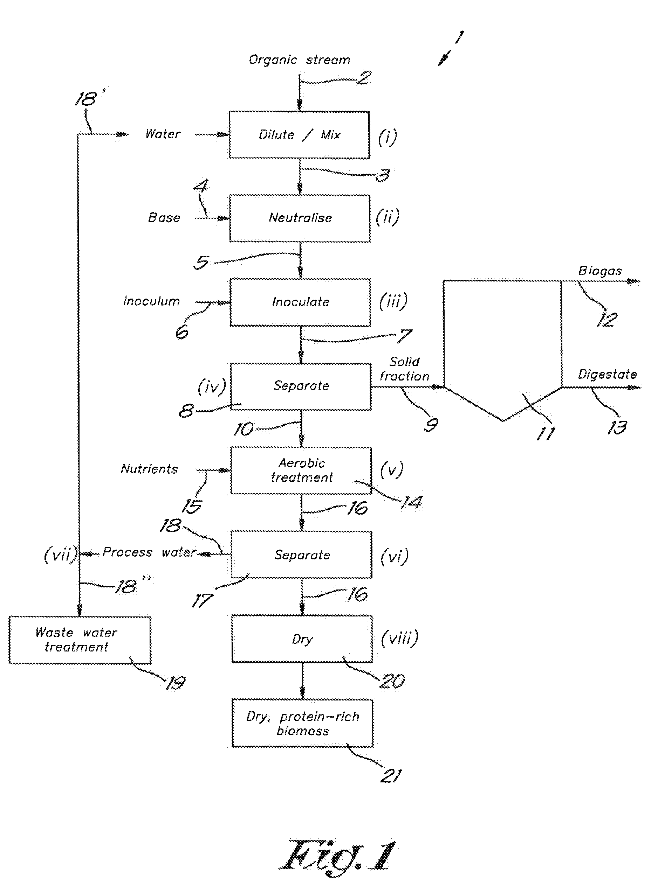

figure 1 schematically shows a flow diagram of the method for the integrated production of bioproducts according to the invention;

Figure 1 schematically shows the process of the method according to the invention for the integrated production of bioproducts from streams of organic material from organic waste or from energy crops, in a flow diagram 1. After mechanical steps, the stream of organic material 2 is diluted and mixed with water in a further step (i) until a dry matter content lower than 15% is reached, after which the mixture 3 can undergo an alkaline treatment in a next step (ii) by adding a base 4 until a pH between 5 and 9 is reached. In a next step (iii) an inoculum 6 can be added to the neutral mixture 5, and this practically directly, consisting of a natural anaerobic culture which stimulates the release of the organic compounds, after which in step (iv) in a first separation step 8 the inoculated mixture 7 is split after a sufficiently long contact time into a solid fraction 9 and a liquid fraction 10. The solid fraction 9 is transported to an anaerobic digestion plant 11 where said fraction is reduced to biogas 12 and digestate 13. The separated liquid fraction 10 from step (iv) undergoes an aerobic treatment 14 in step (v) after addition of nutrients 15, whereby the dissolved organic compounds are biologically converted into a protein-rich bioproduct 16. Subsequently, a second separation step 17 follows in step (vi), this time on the remaining effluent, whereby the formed protein-rich bioproduct 16 is separated from the liquid phase and in step (vii) the remaining process water 18 is partly reutilised as process water 18' in step (i) and partly discharged as 18" to a waste water treatment plant 19. The protein-rich bioproduct 16 from step (vi) further undergoes a drying step 20 in step (viii) to remove the remaining water, after which a dry, protein-rich bioproduct 21 is obtained, with a protein content of 50-80%, in addition to the bioproducts biogas 12 and digestate 13 in one run of the processing plant 1.

## Claims

1. Method for producing bioproducts from streams of organic material, **characterised in that** it at least consists of the following steps:
(i) a physical-biological pre-treatment of the organic stream whereby the substrate is mixed with water into a diluted stream with a dry matter content lower than 15% and one or more mechanical steps are applied such as continuous grinding, blixing and continuous extruding,
(ii) an alkaline treatment by adding an alkaline solution until a pH value of the mixture between pH 5 and pH 9 is reached,
iii) adding an inoculum consisting of a natural anaerobic culture which stimulates the release of the organic compounds,
iv) a first separation step by centrifuging, compressing or filtering which splits the mixture into a solid fraction and a liquid fraction, whereby the solid fraction is further processed via an anaerobic fermentation technology, with the formation of biogas and digestate as end products,
v) a continuous aerobic treatment of the separated liquid fraction whereby the organic compounds are biologically converted into a protein-rich bioproduct, after addition of the necessary nutrients,
vi) a second separation step whereby the formed protein-rich bioproduct is separated from the liquid phase,
vii) recirculating the liquid phase partly as process water to the mixing step (ii) and partly discharged and treated in a waste water purification plant,
viii) drying the formed protein-rich bioproduct to remove the remaining water after which a dry, protein-rich bioproduct is obtained and the bioproducts biogas and digestate are also obtained.

2. Method according to claim 1, **characterised in that** the method is executed in a continuous process, in which a continuous process for anaerobic fermentation is integrated.

3. Method according to claim 1, **characterised in that** when mixing the organic stream with water, possibly with addition of the alkaline solution and/or inoculum, a sufficiently long contact time of five minutes to two weeks must be maintained, more specifically a contact time between five minutes and one hour or between one and six hours or between six hours and one day or between one day and one week, or between one week and two weeks. The temperature imposed during the contact time is between 10 and 80 °C, more specifically between 10 and 25 °C, or between 25 and 40 °C, or between 40 and 60 °C, or between 60 and 80 °C.

## Patentansprüche

1. Verfahren zur Herstellung von Bioprodukten aus Strömen organischen Materials, **dadurch gekennzeichnet, dass** es zumindest aus folgenden Schritten besteht:
(i) eine physikalisch-biologische Vorbehandlung des organischen Stroms, wobei das Substrat mit Wasser gemischt wird, um einen verdünnten Strom zu erhalten, der einen Trockensubstanzgehalt von weniger als 15% aufweist, und wobei ein oder mehrere mechanische Schritte wie kontinuierliches Mahlen, "Blixen" und kontinuierliches Extrudieren angewendet werden,
(ii) eine alkalische Behandlung durch Zugabe einer alkalischen Lösung, bis ein pH-Wert der Mischung zwischen pH 5 und pH 9 erreicht ist;
(iii) das Zugeben eines Inokulums, das aus einer natürlichen anaeroben Kultur besteht, die die Freisetzung der organischen Verbindungen stimuliert;
(iv) einen ersten Trennschritt durch Zentrifugieren, Komprimieren oder Filtern, der das Gemisch in eine feste Fraktion und eine flüssige Fraktion aufspaltet, wobei die feste Fraktion über eine anaerobe Fermentationstechnologie weiterverarbeitet wird, wobei die Bildung von Biogas und Gärrückstand als Endprodukte erhalten wird;
(v) eine kontinuierliche aerobe Behandlung der abgetrennten flüssigen Fraktion, wobei die organischen Verbindungen nach Zugabe der notwendigen Nährstoffe biologisch in ein proteinreiches Bioprodukt umgewandelt werden;
(vi) einen zweiten Trennschritt, wobei das gebildete proteinreiche Bioprodukt von der flüssigen Phase getrennt wird;
(vii) das Rezirkulieren der flüssigen Phase teilweise als Prozesswasser zu dem Mischschritt (ii), und teilweise als abgeleitetes und behandeltes Wasser in einer Kläranlage;
(viii) das Trocknen des gebildeten proteinreichen Bioprodukts, um das verbleibende Wasser zu entfernen, wonach ein trockenes, proteinreiches Bioprodukt erhalten wird, und die Bioprodukte Biogas und Gärrückstand ebenfalls erhalten werden.

2. Verfahren nach Anspruch 1, **dadurch gekennzeichnet, dass** das Verfahren in einem kontinuierlichen Prozess durchgeführt wird, in den ein kontinuierlicher Prozess zur anaeroben Fermentation integriert wird.

3. Verfahren nach Anspruch 1, **dadurch gekennzeichnet, dass** beim Mischen des organischen Stromes mit Wasser, gegebenenfalls unter Zugabe der alkalischen Lösung und/oder des Inokulums, eine ausreichend lange Kontaktzeit von fünf Minuten bis zwei Wochen eingehalten werden muss, genauer gesagt eine Kontaktzeit zwischen fünf Minuten und einer Stunde oder zwischen einer und sechs Stunden oder zwischen sechs Stunden und einem Tag oder zwischen einem Tag und einer Woche oder zwischen einer Woche und zwei Wochen, wobei die während der Kontaktzeit vorgeschriebene Temperatur zwischen 10 und 80 °C, genauer gesagt zwischen 10 und 25 °C, oder zwischen 25 und 40 °C, oder zwischen 40 und 60 °C, oder zwischen 60 und 80 °C liegt.

## Revendications

1. Procédé destiné à la production de bioproduits à partir de flux de matières organiques, **caractérisé en ce qu'**il comprend au moins les étapes suivantes :
(i) un prétraitement physico-biologique du flux organique par lequel le substrat est mélangé avec de l'eau afin d'obtenir un flux dilué dont la teneur en matière sèche est inférieure à 15 %, ainsi que l'application d'une ou de plusieurs étapes mécaniques, telles qu'un broyage en continu, un « blixing » et une extrusion en continu ;
(ii) un traitement alcalin par addition d'une solution alcaline jusqu'à ce que l'on atteigne une valeur de pH du mélange entre un pH de 5 et un pH de 9 ;
(iii) l'addition d'un inoculum constitué d'une culture anaérobie naturelle qui stimule la libération des composés organiques ;
(iv) une première étape de séparation par centrifugation, compression ou filtration qui divise le mélange en une fraction solide et une fraction liquide, dans lequel la fraction solide est soumise à un traitement ultérieur par l'intermédiaire d'une technologie de fermentation anaérobie, avec la formation de biogaz et de digestat à titre de produits finis ;
(v) un traitement aérobie en continu de la fraction liquide séparée, par lequel les composés organiques sont convertis par voie biologique en un bioproduit riche en protéines, après addition des nutriments nécessaires ;
(vi) une deuxième étape de séparation dans laquelle le bioproduit riche en protéines que l'on a obtenu est séparé de la phase liquide ;
(vii) la remise en circulation de la phase liquide en partie sous la forme d'eau de traitement dans l'étape de mélange (ii) et en partie sous forme évacuée et traitée dans une station d'épuration des eaux usées ;
(viii) le séchage du bioproduit riche en protéines que l'on a obtenu, dans le but d'éliminer l'eau résiduelle ; après quoi on obtient un bioproduit sec riche en protéines et on obtient également les bioproduits que sont le biogaz et le digestat.

2. Procédé selon la revendication 1, **caractérisé en ce que** le procédé est mis en oeuvre au cours d'un processus en continu, dans lequel est intégré un processus en continu à des fins de fermentation anaérobie.

3. Procédé selon la revendication 1, **caractérisé en ce que**, au cours du mélange du flux organique avec de l'eau, le cas échéant avec l'addition de la solution alcaline et/ou de l'inoculum, un temps de mise en contact suffisamment long de cinq minutes à deux semaines doit être maintenu, de manière plus spécifique un temps de mise en contact entre cinq minutes et une heure ou entre une et six heures ou entre six heures et un jour ou entre un jour et une semaine ou entre une semaine et deux semaines, dans lequel la température imposée au cours du laps de temps de mis en contact est comprise entre 10 et 80 °C, de manière plus spécifique entre 10 et 25 °C, ou entre 25 et 40 °C, ou entre 40 et 60 °C, ou entre 60 et 80 °C.
